(19) Europäisches Patentamt
European Patent Office
Office européen des brevets

(11) Publication number: 0 056 458
B1

(12) # EUROPEAN PATENT SPECIFICATION

(45) Date of publication of patent specification: 02.05.85

(51) Int. Cl.4: C 07 H 15/04, A 61 K 31/70 // C07H13/12

(21) Application number: 81110217.7

(22) Date of filing: 07.12.81

(54) **Nitrosourea derivatives, a process for preparing same and therapeutic compositions.**

(30) Priority: 19.01.81 JP 6860/81

(43) Date of publication of application:
28.07.82 Bulletin 82/30

(45) Publication of the grant of the patent:
02.05.85 Bulletin 85/18

(84) Designated Contracting States:
BE CH DE FR GB IT LI NL

(56) References cited:
JP-A-55 004 324

CHEMICAL ABSTRACTS, vol. 92, 1980, page 631, no. 147144h Columbus, Ohio, U.S.A.
CHEMICAL ABSTRACTS, vol. 92, 1980, page 660, no. 215731z Columbus, Ohio, U.S.A.

**The file contains technical information submitted after the application was filed and not included in this specification**

(73) Proprietor: Tanabe Seiyaku Co., Ltd.
No. 21 Dosho-machi 3-chome Higashi-ku
Osaka-shi Osaka-fu (JP)

(72) Inventor: Morikawa, Tamio
No. 16-2, Kaminakazato 2-chome
Kita-ku Tokyo-to (JP)
Inventor: Tsujihara, Kenji
No. 1149-133, Oaza-Omaki
Urawa-shi Saitama-ken (JP)
Inventor: Arai, Yoshihisa
No. 17-1, Maechi 3-chome
Urawa-shi Saitama-ken (JP)

(74) Representative: Hansen, Bernd, Dr.rer.nat. et al
Hoffmann, Eitle & Partner Patentanwälte
Arabellastrasse 4
D-8000 München 81 (DE)

EP 0 056 458 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to nitrosourea derivatives, a process for preparing same and therapeutic compositions. More particularly, it relates to compounds of the formula:

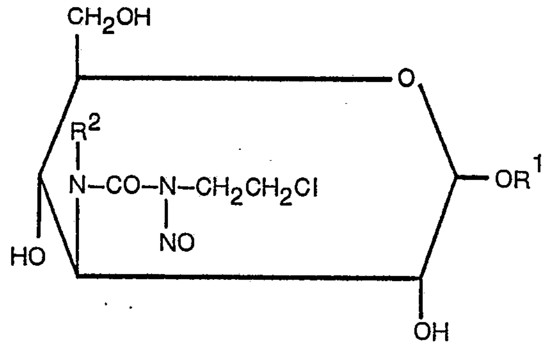

(I)

wherein $R^1$ is straight alkyl having 1 to 5 carbon atoms and $R^2$ is straight or branched alkyl having 1 to 5 carbon atoms, straight alkenyl having 2 to 5 carbon atoms or methoxy-$C_{1-5}$ alkyl.

It is known that 1 - (2 - chloroethyl) - 1 - nitroso - 3 - β - (D - mannopyranosyl)urea and 1 - (2 - chloroethyl) - 1 - nitroso - 3 - β - (D - glucopyranosyl)urea (the latter compound being hereinafter referred to as "GANU") increase the life span of mice implanted intraperitoneally with the tumor cells of lymphoid luekemia L-1210 (U.S. Patent No. 4086415). It is also known that 1 - (2 - chloroethyl) - 1 - nitroso - 3 - β - (D - lactosyl)urea and 1 - (2 - chloroethyl) - 1 - nitroso - 3 - β - (D - maltosyl)urea show anti-tumor activity against leukemic cells (Japanese Patent Publication (unexamined) No. 141815/1976).

In "Chemical Abstracts" Vol. 92 (1980), No. 1471144h a compound III is disclosed as an anti-cancer agent, which differs from compounds according to the present main claim in that $R^1$ and $R^2$ are represented by hydrogen.

"Chemical Abstracts" Vol. 92 (1980), No. 215731z discloses compounds as anti-cancer agents which differ from the compounds according to the invention in that the sugar moiety therein is represented by an alkyl 6-deoxy-aldo-hexopyranosido-6-yl or alkyl - 5 - deoxy - aldopentofuranosido - 5 - yl radical.

Japanese Patent Application (unexamined) No. 4324/1980 discloses glucopyranosidamine derivatives which possess an anti-tumor activity. It could be shown that the compounds according to the citation, especially 3 - [3 - (2 - chloroethyl)ureido] - 3 - deoxy - D - glucopyranose possesses a lower anti-tumor activity as a respective comparative compound according to the invention.

We have now found that the nitrosourea compound (I) of the present invention shows potent anti-tumor or anti-leukemic activity and is useful to inhibit the growth of malignant tumor cells in warm-blooded animals. For example, when the anti-tumor effects upon leukemia is estimated by administering such drugs intraperitoneally to tumor cell-inoculated mice (i.e., mice implanted with tumor cells of leukemia L-1210) for five consecutive days, 1 - (2 - chloroethyl) - 1 - nitroso - 3 - methyl - 3 - (methyl 3 - deoxy - α - D - glucopyranosid - 3 - yl)urea at the daily dose of 0.26 mg/kg shows an increase of about 30% in the average life span of said mice. Moreover, the compound (I) may show an excellent therapeutic index estimated in terms of ratio of M. T. D. (the maximum tolerated dose which shows 100% inhibition for the growth of Ehrlich ascites tumor in mice without causing the death of said mice) to M. E. D. (the minimum effective dose which shows 100% inhibition for the growth of said ascites tumor). For example, said therapeutic indexes (M. T. D./M. E. D.) of 1 - (2 - chloroethyl) - 1 - nitroso - 3 - isobutyl - 3 - (methyl 3 - deoxy - α - D - glucopyranosid - 3 - yl)urea, 1 - (2 - chloroethyl) - 1 - nitroso - 3 - allyl - 3 - (methyl 3 - deoxy - α - D - glucopyranosid - 3 - yl)urea and 1 - (2 - chloroethyl) - 1 - nitroso - 3 - (2 - methoxyethyl) - 3 - (methyl 3 - deoxy - α - D - glucopyranosid - 3 - yl/urea are 4 times greater than that of GANU.

In the above-mentioned formule (I), representative examples of the group $R^1$ include straight alkyl such as methyl, ethyl, n-propyl and n-butyl. On the other hand, representative examples of the group $R^2$ include straight or branched alkyl such as methyl, ethyl, n-propyl, isopropyl, n-butyl, isobutyl, sec.-butyl, t-butyl, n-amyl, isoamyl, sec-amyl, and t-amyl; straight alkenyl such as vinyl, allyl, butenyl and pentenyl; and methoxy-$C_{1-5}$ alkyl, such as methoxymethyl, methoxyethyl, methoxy-n-propyl, methoxy-n-butyl, methoxy-n-pentyl.

Further, among those compounds of the invention, a preferred sub group includes the compounds of the formula (I) in which $R^1$ is methyl, and $R^2$ is methyl, n-butyl, isobutyl, allyl and methoxyethyl.

According to the present invention, the nitrosourea compound (I) is prepared by nitrosation of a compound of the formula:

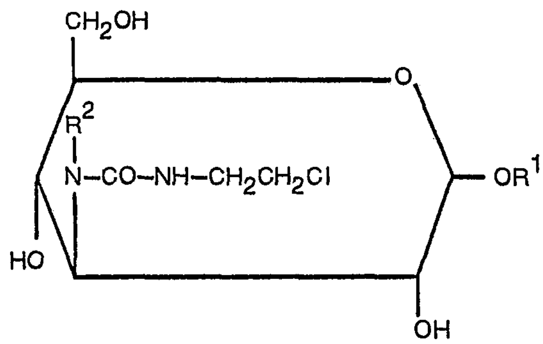

(II)

wherein $R^1$ and $R^2$ are the same as defined above.

The starting compound (II) is readily obtained. For example, it can be prepared by condensing a compound of the formula:

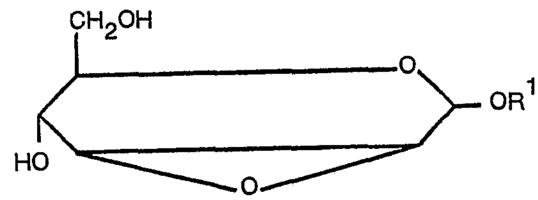

(III)

wherein $R^1$ is the same as defined above, with a primary amine of the formula: $R^2$—$NH_2$ (wherein $R^2$ is the same as defined above) at 120 to 180°C to give a compound of the formula:

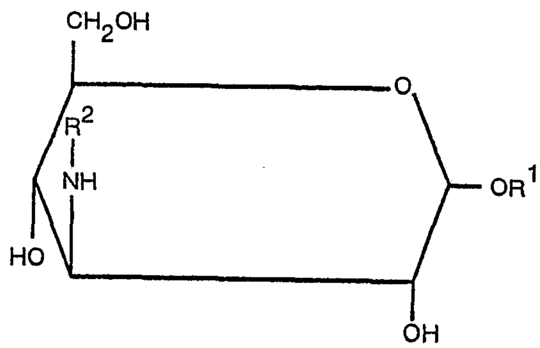

(IV)

wherein $R^1$ and $R^2$ are the same as defined above, and then condensing the compound (IV) with 2-chloroethyl isocyanate at 0 to 25°C in an inert solvent (e.g., methanol, ethanol, tetrahydrofuran). If required, the condensation of the compound (III) and the primary amine may be carried out in an inert solvent such as methanol or ethanol.

The nitrosation reaction of the present invention is accomplished by contacting the compound (II) with nitrous acid, nitrogen trioxide or nitrogen tetroxide in an inert solvent. The reaction can be preferably carried out at a temperature of −20 to 20°C, especially at about −10 to 0°C. Water, tetrahydrofuran, methylene chloride, ethyl acetate, acetic acid, and formic acid are suitable as the inert solvent. When free nitrous acid is prepared by reacting an alkali metal salt of nitrous acid (e.g., sodium nitrite, potassium nitrite) or a lower alkyl ester thereof (e.g., butyl nitrite, amyl nitrite) with a mineral or organic acid (e.g., hydrochloric acid, sulfuric acid, formic acid or acetic acid), it is preferred that said free nitrous acid is employed for subsequent nitrosation reaction immediately after preparation thereof. On the other hand, when nitrogen trioxide or nitrogen tetroxide is employed in the invention, it is preferred to carry out the nitrosation reaction by dissolving or suspending the starting compound (II) in the inert solvent and then introducing gaseous nitrogen trioxide or tetroxide thereto in presence or absence of an acid acceptor. Sodium bicarbonate, sodium carbonate, potassium carbonate, sodium acetate, potassium acetate are suitable as the acid acceptors.

When the nitrosation reaction is completed, the compound (I) of the invention is readily recovered from the reaction mixture and may be, if required, further purified by silica gel chromatography.

The nitrosourea compound (I) thus obtained shows potent anti-tumor activity against various tumor cells such as Ehrlich's carcinoma, Sarcoma 180, Leukemia L-1210, Lewis lung carcinoma, Yoshida sarcoma and Rat ascites hepatoma. It may be useful to prolong the survival time of warm-blooded animals afflicted with said tumors and/or minimize the growth of said tumors in said animals. It may also be employed for therapy of malignant lymphoma, leukemia, stomach tumor and hepatoma. The nitrosourea compound (I) can be used for pharmaceutical use in the form of a pharmaceutical preparation suitable for either oral or parenteral administration. The compound (I) may also be used in conjunction or admixture with a

pharmaceutical excipient. The excipient selected must be the one which does not react with the compound (I). Suitable excipients include, for example, gelatin, lactose, glucose, sodium chloride, starch, magnesium stearate, talcum, vegetable oil and so forth. Other known medicinal excipients may be employed. The pharmaceutical preparation may be a solid dosage form such as, for example, a tablet, a coated tablet, a pill or a capsule; or a liquid dosage form such as, for example, a solution, a suspension or an emulsion. Further, the compound (I) may be employed, for example, in the form of an injection or suppository when administered parenterally. The pharmaceutical preparation may be sterilized and/or may contain auxiliaries such as, for example, preserving and stabilizing agents. The dose of the compound (I) for pharmaceutical use depends on route of administration; the age, weight and condition of the patients; and particular diseases to be treated. In general, it may be used for pharmaceutical use at a dose of 0.1 to 15 mg/kg, especially 0.2 to 5 mg/kg, per day.

Experiments

Chemotherapeutic effects of the nitrosourea compounds of the invention on tumor cells in mice were investigated by the following methods and materials.

(Methods)

(A) Preventive effect against the growth of Ehrlich ascites tumor:

$10^6$ tumor cells of Ehrlich ascites carcinoma were inoculated intraperitoneally into a group of five female mice (ICR mice, body weight: 19—23 g). A test compound was dissolved in a physiological saline solution. (In the case where CCNU was employed as the test compound, said compound was suspended in a physiological saline solution containing 0.1% NIKKOL HCO-60 (trademark; a surface active agent manufactured by Nikko Chemicals Co. Ltd.)) and administered intraperitoneally to the mice. The administration of the test compound was begun 24 hours after the inoculation of the tumor cells and performed once a day for 5 days. The volume of ascites in the treated mice were measured after 7 days of the experiment.

(B) Effect on the life span of mice implanted with leukemic cells of L-1210:

$10^5$ leukemic cells of L-1210 were inoculated intraperitoneally into a group of four male mice ($BDF_1$ mice, body weight: 19—23 g). A test compound was dissolved in a physiological saline solution and administered intraperitoneally to the mice. The administration of the test compound was begun 24 hours after the inoculation of the leukemic cells and performed once a day for 5 days. The survival days of the treated mice were observed.

(Compound tested)

Compound nos. chemical names

(The compounds of the present invention)

1. 1 - (2 - chloroethyl) - 1 - nitroso - 3 - n - butyl - 3 - (methyl 3 - deoxy - α - D - glucopyranosid - 3 - yl)urea
2. 1 - (2 - chloroethyl) - 1 - nitroso - 3 - isobutyl - 3 - (methyl 3 - deoxy - α - D - glucopyranosid - 3 - yl)urea
3. 1 - (2 - chloroethyl) - 1 - nitroso - 3 - methyl - 3 - (methyl 3 - deoxy - α - D - glucopyranosid - 3 - yl)urea
4. 1 - (2 - chloroethyl) - 1 - nitroso - 3 - allyl - 3 - (methyl 3 - deoxy - α - D - glucopyranosid - 3 - yl)urea
5. 1 - (2 - chloroethyl) - 1 - nitroso - 3 - propargyl - 3 - (methyl 3 - deoxy - α - D - glucopyranosid - 3 - yl)urea
6. 1 - (2 - chloroethyl) - 1 - nitroso - 3 - (2 - methoxyethyl) - 3 - (methyl 3 - deoxy - α - D - glucopyranosid - 3 - yl)urea

(Known compounds)

CCNU: 1-(2-chloroethyl)-1-nitroso-3-cyclohexylurea

GANU: 1-(2-chloroethyl)-1-nitroso-3-β-(D-glucopyranosyl)urea

(Results)

The results of the experiments are shown in the following Tables 1 and 2.

TABLE 1
Preventive effect against the growth of Ehrlich ascites carcinoma (method A)

| Compound Nos. | Dose (mg/kg/day) | Ascites volume (g) T/C[a] | Inhibition ratio[b] (%) | MTD[c] | MED[d] | Therapeutic index[e] |
|---|---|---|---|---|---|---|
| | 100 | — | Toxic (4/5)* | | | |
| | 50 | 0.0/4.2 | 100 | | | |
| | 12.5 | 0.0/4.2 | 100 | | | |
| 1. | 3.12 | 0.0/4.2 | 100 | 50 | 0.78 | 64 |
| | 0.78 | 0.0/4.2 | 100 | | | |
| | 0.39 | 1.7/4.2 | 59.5 | | | |
| | 0.19 | 3.6/4.2 | 14.3 | | | |
| | 50 | — | Toxic (1/5)* | | | |
| | 25 | 0.0/3.7 | 100 | | | |
| | 6.25 | 0.0/3.7 | 100 | | | |
| 2. | 1.56 | 0.0/3.7 | 100 | 25 | 0.195 | 128 |
| | 0.39 | 0.0/3.7 | 100 | | | |
| | 0.195 | 0.0/3.7 | 100 | | | |
| | 0.097 | 3.4/3.7 | 8.1 | | | |
| | 50 | — | Toxic (1/5)* | | | |
| | 25 | 0.0/3.7 | 100 | | | |
| | 6.25 | 0.0/3.7 | 100 | | | |
| 3. | 1.56 | 0.0/3.7 | 100 | 25 | 0.39 | 64 |
| | 0.39 | 0.0/3.7 | 100 | | | |
| | 0.195 | 0.7/3.7 | 81.1 | | | |
| | 0.097 | 3.6/3.7 | 2.7 | | | |
| | 50 | — | Toxic (3/5)* | | | |
| | 25 | 0.0/3.7 | 100 | | | |
| | 6.25 | 0.0/3.7 | 100 | | | |
| 4. | 1.56 | 0.0/3.7 | 100 | 25 | 0.195 | 128 |
| | 0.39 | 0.0/3.7 | 100 | | | |
| | 0.195 | 0.0/3.7 | 100 | | | |
| | 0.097 | 2.6/3.7 | 29.7 | | | |

TABLE 1 (continued)

| Compound Nos. | Dose (mg/kg/day) | Ascites volume (g) T/C[(a)] | Inhibition ratio[(b)] (%) | MTD[(c)] | MED[(d)] | Therapeutic index[(e)] |
|---|---|---|---|---|---|---|
| 5. | 50 | — | Toxic (1/5)* | 25 | 0.78 | 32 |
| | 25 | 0.0/3.7 | 100 | | | |
| | 6.25 | 0.0/3.7 | 100 | | | |
| | 1.56 | 0.0/3.7 | 100 | | | |
| | 0.78 | 0.0/3.7 | 100 | | | |
| | 0.39 | 2.4/3.7 | 35.1 | | | |
| 6. | 50 | — | Toxic (2/5)* | 25 | 0.195 | 128 |
| | 25 | 0.0/3.7 | 100 | | | |
| | 6.25 | 0.0/3.7 | 100 | | | |
| | 1.56 | 0.0/3.7 | 100 | | | |
| | 0.39 | 0.0/3.7 | 100 | | | |
| | 0.195 | 0.0/3.7 | 100 | | | |
| | 0.097 | 3.7/3.7 | 0.0 | | | |
| CCNU | 100 | — | Toxic (5/5)* | 50 | 12.5 | 4 |
| | 50 | 0.0/5.7 | 100 | | | |
| | 12.5 | 0.0/5.7 | 100 | | | |
| | 6.25 | 3.8/5.7 | 33.3 | | | |
| | 3.12 | 4.5/5.7 | 21.1 | | | |
| GANU | 25 | — | Toxic (5/5)* | 12.5 | 0.39 | 32 |
| | 12.5 | 0.0/4.8 | 100 | | | |
| | 3.12 | 0.0/4.8 | 100 | | | |
| | 0.78 | 0.0/4.8 | 100 | | | |
| | 0.39 | 0.0/4.8 | 100 | | | |
| | 0.19 | 1.0/4.8 | 79.2 | | | |
| | 0.09 | 4.6/4.8 | 4.2 | | | |

Note:

(a): T=the average volume of ascites in the treated mice
C=the average volume of ascites in the untreated mice (control group of mice)

(b): Inhibition ratio $(\%)=\frac{C-T}{C}\times 100$

(c): MTD=Maximum Tolerated Dose (i.e., the maximum dose which shows 100% inhibition for the growth of Ehrlich ascites tumor in mice without causing the death of said mice)

(d): MED=Minimum Effective Dose (i.e., the minimum dose which shows 100% inhibition for the growth of said ascites tumor)
(e): Therapeutic index=MTD/MED
*: the number of mice died/the number of mice used

TABLE 2
Effect of life span of mice implanted with Leukemia L-1210 (method B)

| Compound No. | Dose (mg/kg/day) | Mean survival days (T/C) (a) | ILS (%) (b) | 60-day survivors (c) |
|---|---|---|---|---|
| 1. | 25 | >60.0/7.1 | >745.1 | 4/4 |
| | 12.5 | >60.0/7.1 | >745.1 | 4/4 |
| | 6.25 | 15.0/7.1 | 111.3 | 0/4 |
| 2. | 12.5 | >60.0/8.6 | >597.7 | 4/4 |
| | 6.25 | >60.0/8.6 | >597.7 | 4/4 |
| | 3.12 | 13.5/8.6 | 57.0 | 0/4 |
| 3. | 12.5 | >60.0/8.4 | >614.3 | 4/4 |
| | 6.25 | >60.0/8.4 | >614.3 | 4/4 |
| | 3.12 | >38.8/8.4 | >361.9 | 2/4 |
| | 1.56 | 14.3/8.4 | 70.2 | 0/4 |
| 4. | 25 | >51.3/8.6 | >496.5 | 3/4 |
| | 12.5 | >60.0/8.6 | >597.7 | 4/4 |
| | 6.25 | 15.5/8.6 | 80.2 | 0/4 |

Note:
(a): T=the mean survival days of the treated mice
C=the mean survival days of the untreated mice (control group of mice)

(b): ILS (Increase in Life Span)=$\frac{T-C}{C}\times 100$

(c): 60-day survivors=the number of mice survived for 60 days/the number of mice used

Practical and presently-preferred embodiments of the present invention are illustratively shown in the following Examples.

Example 1

(1) 2.5 g of methyl 2,3-anhydro-α-allopyranoside (J. Chem. Soc., 472 (1928)), 10 g of n-butylamine and 20 ml of ethanol are heated at 150°C for 30 hours in a sealed tube. The reaction mixture is concentrated under reduced pressure to dryness, whereby methyl 3-n-butylamino-3-deoxy-α-D-glucopyranoside is obtained as a crude product. Said crude product is dissolved in 40 ml of methanol, and 2.5 g of 2-chloroethyl isocyanate are added dropwise thereto at a temperature of 0 to 5°C. The mixture is stirred at room temperature for 1.5 hours. After the reaction, the mixture is evaporated under reduced pressure to remove solvent. The residue is purified by silica gel chromatography (Solvent; chloroform: benzene:methanol=5:2:1), whereby 3.0 g of 1 - (2 - chloroethyl) - 3 - n - butyl - 3 - (methyl 3 - deoxy - α - D - glucopyranosid - 3 - yl)urea are obtained as colorless syrup.

$[\alpha]_D^{25}$+106.5° (C=1.23, methanol)

IR$\nu_{max.}^{Nujol}$ ($cm^{-1}$): 3400, 1670, 1615, 1520, 1040

NMR ($d_6$-DMSO) δ: 0.80—1.90 (m, 7H, —$CH_2CH_2CH_2CH_2$), 3.32 (s, 3H, $OCH_3$), 4.64 (d, 1H, $H_1$)

(2) 1.8 g of 1 - (2 - chloroethyl) - 3 - n - butyl - 3 - (methyl 3 - deoxy - α - D - glucopyranosid - 3 - yl)urea and one g of anhydrous sodium acetate are suspended in 40 ml of ethyl acetate, and 2.2 g of nitrogen tetroxide gas are introduced into the suspension at −10°C for 10 minutes under stirring. The mixture is stirred at same temperature for 20 minutes. 6 ml of methanol are added to the reaction mixture and said mixture is further stirred for about 10 minutes. The reaction mixture is adjusted to pH 5 to 6 with an aqueous 20% sodium carbonate solution. The organic layer is collected from the mixture, dried and evaporated under reduced pressure to remove solvent. The residue is purified by silica gel chromatography (Solvent; ethyl acetate: benzene:methanol=8:2:1), whereby 1.1 g of 1 - (2 - chloroethyl) - 1 - nitroso - 3 - n - butyl - 3 - (methyl 3 - deoxy - α - D - glucopyranosid - 3 - yl)urea are obtained as yellow syrup.

M.p. 50°C (decomp.)

$[\alpha]_D^{25}$+90.7° (C=1.08, methanol)

$IR\nu_{max.}^{Nujol}$ ($cm^{-1}$): 3450, 1680, 1040

NMR ($d_6$-DMSO) δ: 0.70—1.80 (m, 7H, —$CH_2CH_2CH_2CH_2$), 3.32 (s, 3H, $OCH_2$), 4.62 (d, 1H, J=3Hz, $H_1$)

Example 2

(1) 2.5 g of methyl 2,3-anhydro-α-allopyranoside, 10 g of isobutylamine and 2.5 g of 2-chloroethyl isocyanate are treated in the same manner as described in Example 1-(1), whereby 2.7 g of 1 - (2 - chloroethyl) - 3 - isobutyl - 3 - (methyl 3 - deoxy - α - D - glucopyranosid - 3 - yl)urea are obtained as colorless syrup.

$[\alpha]_D^{21}$+63.4° (C=1.54, methanol)

$IR\nu_{max.}^{Nujol}$ ($cm^{-1}$): 3300, 1660, 1560, 1510, 1100, 1040

NMR ($d_6$-DMSO) δ: 0.88 (d, d, 6H, J=6Hz, —$CH(CH_3)_2$, 3.33 (s, 3H, $OCH_3$), 4.68 (d, 1H, J=3Hz, $H_1$)

(2) 1.8 g of 1 - (2 - chloroethyl) - 3 - isobutyl - 3 - (methyl 3 - deoxy - α - D - glucopyranosid - 3 - yl)urea and 2.2 g of nitrogen tetroxide gas are treated in the same manner as described in Example 1-(2), whereby 0.9 g of 1 - (2 - chloroethyl) - 1 - nitroso - 3 - isobutyl - 3 - (methyl 3 - deoxy - α - D - glucopyranosid - 3 - yl)urea is obtained as yellow syrup.

M.p. 53°C (decomp.)

$[\alpha]_D^{18}$+84.2° (C=1.22, methanol)

$IR\nu_{max.}^{Nujol}$ ($cm^{-1}$): 3440, 1690, 1070, 1040, 1010

NMR ($d_6$-DMSO) δ: 0.89 (d, 6H, J=6.5Hz, —$CH(CH_3)_2$), 3.30 (s, 3H, $OCH_3$), 4.64 (d, 1H, J=2.5Hz, $H_1$)

Example 3

(1) 5.2 g of methyl 2,3-anhydro-α-allopyranoside, 30 ml of 30% methylamine-ethanol solution and 4.5 g of 2-chloroethyl isocyanate are treated in the same manner as described in Example 1-(1), whereby 1.6 g of 1-(2-chloroethyl)-3-methyl-3-(methyl 3-deoxy-α-D-glucopyranosid-3-yl)urea are obtained as colorless syrup.

$[\alpha]_D^{18}$+123.7° (C=0.88, methanol)

NMR ($d_6$-DMSO) δ: 3.00 (s, 3H, —N—$CH_3$), 3.32 (s, 3H, $OCH_3$), 4.98 (d, 1H J=3Hz, $H_1$), 6.52 (broad, 1H, —NH—)

(2) 1.6 g of 1 - (2 - chloroethyl) - 3 - methyl - 3 - (methyl 3 - deoxy - α - D - glucopyranosid - 3 - yl)urea and 2.2 g of nitrogen tetroxide gas are treated in the same manner as described in Example 1-(2), whereby 0.9 g of 1 - (2 - chloroethyl) - 1 - nitroso - 3 - methyl - 3 - (methyl 3 - deoxy - α - D - glucopyranosid - 3 - yl)urea are obtained as yellow syrup.

M.p. 63°C (decomp.)

$[\alpha]_D^{20}$+99.0° (C=1.38, methanol)

$IR\nu_{max.}^{Nujol}$ ($cm^{-1}$): 3400, 1710, 1545, 1080, 1055, 1030

NMR ($d_6$-DMSO) δ: 3.00 (s, 3H, —N—$CH_3$), 3.48 (s, 3H, $OCH_3$), 4.96 (d, 1H, J=2.5Hz, $H_1$)

Example 4

(1) 2.5 g of methyl 2,3-anhydro-α-allopyranoside, 10 g of allylamine and 2.5 g of 2-chloroethyl isocyanate are treated in the same manner as described in Example 1-(1), whereby 2.1 g of 1 - (2 - chloroethyl) - 3 - allyl - 3 - (methyl 3 - deoxy - α - D - glucopyranosid - 3 - yl)urea are obtained as colorless syrup.

$[\alpha]_D^{21}$+102.3° (C=1.09, methanol)

$IR\nu_{max.}^{Nujol}$ ($cm^{-1}$): 3300, 1670, 1640, 1510, 1040

NMR ($d_6$-DMSO) δ: 3.32 (s, 3H, $OCH_3$), 4.67 (d, 1H, J=3Hz, $H_1$), 6.17 (broad, 1H, —NH—)

(2) 1.7 g of 1-(2-chloroethyl)-3-allyl-3-(methyl 3-deoxy-α-D-glucopyranosid-3-yl)urea and 2.2 g of nitrogen tetroxide gas are treated in the same manner as described in Example 1-(2), whereby 0.9 g of 1 - (2 - chloroethyl) - 1 - nitroso - 3 - allyl - 3 - (methyl 3 - deoxy - α - D - glucopyranosid - 3 - yl)urea is obtained as yellow syrup.

M.p. 52°C (decomp.)
$[\alpha]_D^{18}$+90.4° (C=1.28, methanol)
$IR\nu_{max.}^{Nujol}$ ($cm^{-1}$): 3450, 1690, 1050, 1010
NMR ($d_6$-DMSO) δ: 3.33 (s, 3H, $OCH_3$), 4.64 (d, 1H J=3Hz, $H_1$)

Example 5

(1) 2.5 g of methyl 2,3-anhydro-α-allopyranoside, 10 g of propargylamine and 2.5 g of 2-chloroethyl isocyanate are treated in the same manner as described in Example 1-(1), whereby 3.0 g of 1 - (2 - chloroethyl) - 3 - propargyl - 3 - (methyl 3 - deoxy - α - D - glucopyranosid - 3 - yl)urea are obtained as colorless syrup.
$[\alpha]_D^{21}$+85.6° (C=1.37, methanol)
$IR\nu_{max.}^{Nujol}$ ($cm^{-1}$): 3280, 1670, 1640, 1520, 1040
NMR ($d_6$-DMSO) δ: 3.19 (s, 1H, —C C*H*), 3.34 (s, 3H, $OCH_3$), 4.63 (d, 1H, J=2Hz, $H_1$), 6.54 (broad, 1H, —N*H*—)

(2) 1.7 g of 1 - (2 - chloroethyl) - 3 - propargyl - 3 - (methyl 3 - deoxy - α - D - glucopyranosid - 3 - yl)urea and 2.2 g of nitrogen tetroxide gas are treated in the same manner as described in Example 1-(2), whereby 0.8 g of 1 - (2 - chloroethyl) - 1 - nitroso - 3 - propargyl - 3 - (methyl 3 - deoxy - α - D - glucopyranosid - 3 - yl)urea is obtained as yellow syrup.
M.p. 50°C (decomp.)
$[\alpha]_D^{18}$+81.4° (C=1.02, methanol)
$IR\nu_{max.}^{Nujol}$ ($cm^{-1}$): 3450, 1690, 1070, 1040, 1010
NMR ($d_6$-DMSO) δ: 3.32 (s, 3H, $OCH_3$), 4.66 (d, 1H, J=3Hz, $H_1$)

Example 6

(1) 2.5 g of methyl 2,3-anhydro-α-allopyranoside, 10 g of 2-methoxyethylamine and 2.5 g of 2-chloroethyl isocyanate are treated in the same manner as described in Example 1-(1), whereby 3.4 g of 1 - (2 - chloroethyl) - 3 - (2 - methoxyethyl) - 3 - (methyl 3 - deoxy - α - D - glucopyranosid - 3 - yl)urea are obtained as colorless syrup.
$[\alpha]_D^{21}$+49.8° (C=1.38, methanol)
$IR\nu_{max.}^{Nujol}$ ($cm^{-1}$): 3300, 1670, 1625, 1590, 1515, 1040
NMR ($d_6$-DMSO) δ: 3.24 (s, 3H, $OCH_3$), 3.34 (s, 3H, $OCH_3$), 6.29 (broad, 1H, —N*H*—)

(2) 1.8 g of 1 - (2 - chloroethyl) - 3 - (2 - methoxyethyl) - 3 - (methyl 3 - deoxy - α - D - glucopyranosid - 3 - yl)urea and 2.2 g of nitrogen tetroxide gas are treated in the same manner as described in Example 1-(2), whereby 0.8 g of 1 - (2 - chloroethyl) - 1 - nitroso - 3 - (2 - methoxyethyl) - 3 - (methyl 3 - deoxy - α - D - glucopyranosid - 3 - yl)urea is obtained as yellow syrup.
M.p. 55°C (decomp.)
$[\alpha]_D^{18}$+105.6° (C=1.03, methanol)
$IR\nu_{max.}^{Nujol}$ ($cm^{-1}$): 3400, 1690, 1040, 1010
NMR ($d_6$-DMSO) δ: 3.23 (s, 3H, $OCH_3$), 3.33 (s, 3H, $OCH_3$), 4.65 (d, 1H, J=2.5Hz, $H_1$)

Example 7

(1) 2.5 g of methyl 2,3-anhydro-α-allopyranoside, 10 g of 3-methoxy-n-propylamine and 2.5 g of 2-chloroethyl isocyanate are treated in the same manner as described in Example 1-(1), whereby 4.0 g of 1 - (2 - chloroethyl) - 3 - (3 - methoxy - n - propyl) - 3 - (methyl 3 - deoxy - α - D - glucopyranosid - 3 - yl)urea are obtained as colorless syrup.
$[\alpha]_D^{19}$+76.3° (C=1.3, methanol)
$IR\nu_{max.}^{Nujol}$ ($cm^{-1}$): 3280, 1670, 1045
NMR ($d_6$-DMSO) δ: 1.47—2.10 (m, 2H, —$CH_2CH_2CH_2$—), 3.23 (s, 3H, $OCH_3$), 3.33 (s, 3H, $OCH_3$), 4.65 (d, 1H, J=3Hz, $H_1$), 6.3 (broad, 1H, —N*H*—)

(2) 1.9 g of 1 - (2 - chloroethyl) - 3 - (3 - methoxy - n - propyl) - 3 - (methyl 3 - deoxy - α - D - glucopyranosid - 3 - yl)urea and 2.2 g of nitrogen tetroxide gas are treated in the same manner as described in Example 1-(2), whereby 0.7 g of 1 - (2 - chloroethyl) - 1 - nitroso - 3 - (3 - methoxy - n - propyl) - 3 - (methyl 3 - deoxy - α - D - glucopyranosid - 3 - yl)urea is obtained as yellow syrup.
M.p. 50°C (decomp.)
$[\alpha]_D^{18}$+196.9° (C=1.05, methanol)
$IR\nu_{max.}^{Nujol}$ ($cm^{-1}$): 3400, 1680, 1040
NMR ($d_6$-DMSO) δ: 1.65—2.10 (m, 2H, —$CH_2CH_2CH_2$—), 3.21 (s, 3H, $OCH_3$), 3.33 (s, 3H, $OCH_3$), 4.65 (d, 1H, J=3Hz, $H_1$)

**Claims**

1. Nitrosourea derivative of the formula:

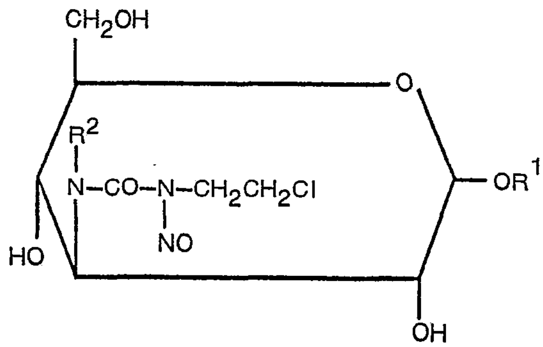

(I)

wherein $R^1$ is straight alkyl having one to five carbon atoms and $R^2$ is straight or branched alkyl having one to five carbon atoms, straight alkenyl having two to five carbon atoms or methoxy-$C_{1-5}$alkyl.

2. The compound of Claim 1, in which $R^1$ is methyl.

3. The compound of Claim 1, in which $R^2$ is methyl, n-butyl, isobutyl, allyl or methoxyethyl.

4. The compound of Claim 1, 2 or 3, in which $R^1$ is methyl and $R^2$ is methyl, n-butyl, isobutyl, allyl or methoxyethyl.

5. The compound of Claim 4, in which $R^1$ is methyl and $R^2$ is methyl.

6. The compound of Claim 4, in which $R^1$ is methyl and $R^2$ is isobutyl.

7. The compound of Claim 4, in which $R^1$ is methyl and $R^2$ is methoxyethyl.

8. A process for preparing a compound of the formula:

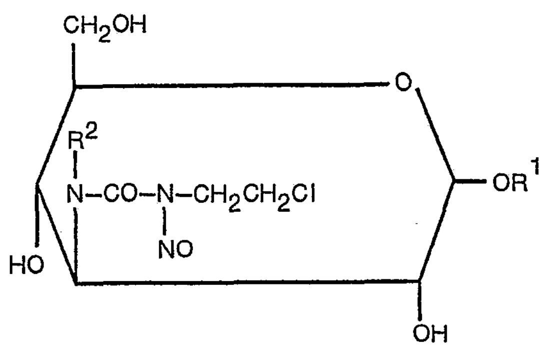

(I)

wherein $R^1$ is straight alkyl having one to five carbon atoms and $R^2$ is straight or branched alkyl having one to five carbon atoms, straight alkenyl having two to five carbon atoms or methoxy-$C_{1-5}$alkyl, which comprises subjecting a compound of the formula:

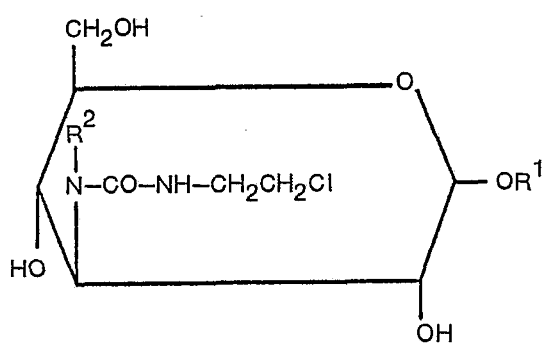

(II)

wherein $R^1$ and $R^2$ are the same as defined above, to nitrosation reaction.

9. The process according to Claim 8, wherein said nitrosation reaction is carried out by contacting the compound (II) with nitrous acid, nitrogen trioxide or nitrogen tetroxide at −20° to 20°C in an inert solvent.

10. A therapeutic composition which comprises a therapeutically effective amount of a compound of the formula:

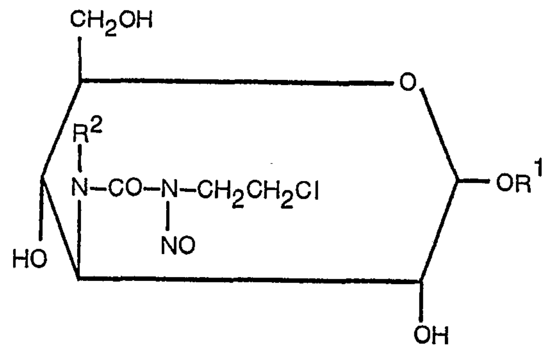

(I)

wherein $R^1$ is straight alkyl having one to five carbon atoms and $R^2$ is straight or branched alkyl having one to five carbon atoms, straight alkenyl having two to five carbon atoms or methoxy-$C_{1-5}$alkyl, and a pharmaceutically acceptable carrier therefor.

**Patentansprüche**

1. Nitrosoharnstoffderivat der Formel:

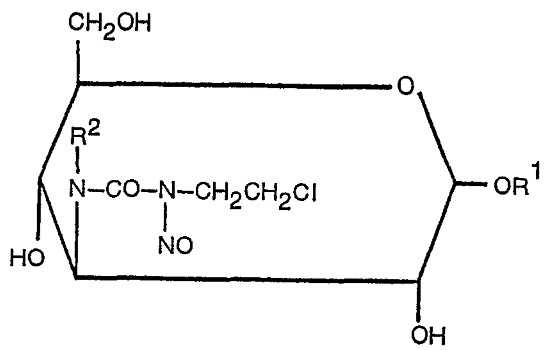

(I)

worin $R^1$ ein lineares Alkyl mit 1 bis 5 Kohlenstoffatomen bedeutet und $R^2$ ein lineares oder verzweigtes Alkyl mit 1 bis 5 Kohlenstoffatomen, lineares Alkenyl mit 2 bis 5 Kohlenstoffatomen oder Methoxy-$C_{1-5}$Alkyl darstellt.

2. Verbindung nach Anspruch 1, worin $R^1$ Methyl bedeutet.

3. Verbindung nach Anspruch 1, worin $R^2$ Methyl, n-Butyl, Isobutyl, Allyl oder Methoxyethyl bedeutet.

4. Verbindung nach Anspruch 1, 2 oder 3, worin $R^1$ Methyl bedeutet und $R^2$ Methyl, n-Butyl, Isobutyl, Allyl oder Methoxyethyl darstellt.

5. Verbindung nach Anspruch 4, worin $R^1$ Methyl und $R^2$ Methyl bedeuten.

6. Verbindung nach Anspruch 4, worin $R^1$ Methyl und $R^2$ Isobutyl bedeuten.

7. Verbindung nach Anspruch 4, worin $R^1$ Methyl und $R^2$ Methoxyethyl bedeuten.

8. Verfahren zur Herstellung einer Verbindung der Formel:

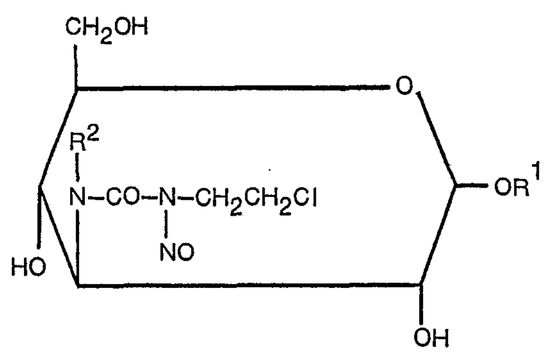

(I)

worin $R^1$ ein lineares Alkyl mit 1 bis 5 Kohlenstoffatomen bedeutet und $R^2$ ein lineares oder verzweigtes Alkyl mit 1 bis 5 Kohlenstoffatomen, lineares Alkenyl mit 2 bis 5 Kohlenstoffatomen oder Methoxy-$C_{1-5}$-Alkyl darstellt, dadurch gekennzeichnet, daß man eine Verbindung der Formel:

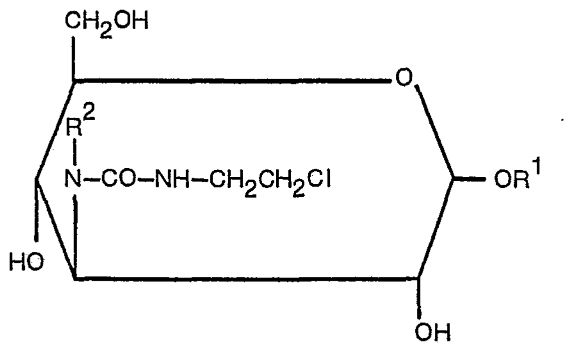

(II)

worin $R^1$ und $R^2$ die vorstehend angegebenen Bedeutungen haben, einer Nitrosierungsreaktion unterwirft.

9. Verfahren nach Anspruch 8, dadurch gekennzeichnet, daß die genannte Nitrosierungsreaktion ausgeführt wird, indem man die Verbindung (II) mit salpetriger Säure, Stickstofftrioxid oder Stickstofftetroxid bei −20 bis 20°C in an einem inerten Lösungsmittel in Kontakt bringt.

10. Therapeutische Zubereitung, dadurch gekennzeichnet, daß sie eine therapeutisch wirksame Menge einer Verbindung der Formel:

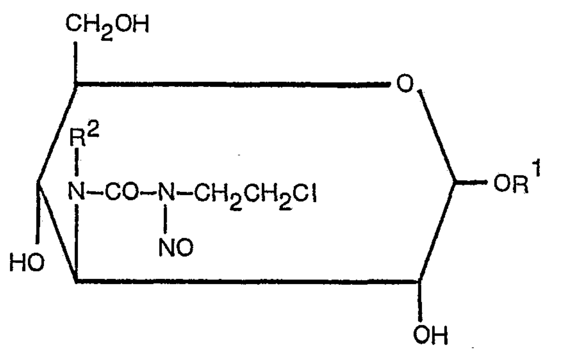

(I)

worin $R^1$ lineares Alkyl mit 1 bis 5 Kohlenstoffatomen bedeutet und $R^2$ lineares oder verzweigtes Alkyl mit 1 bis 5 Kohlenstoffatomen, lineares Alkenyl mit 2 bis 5 Kohlenstoffatomen oder Methoxy-$C_{1-5}$-Alkyl darstellt, und einen pharmazeutisch annehmbaren Träger für dieselbe umfaßt.

**Revendications**

1. Dérivé de nitrosourée ayant la formule:

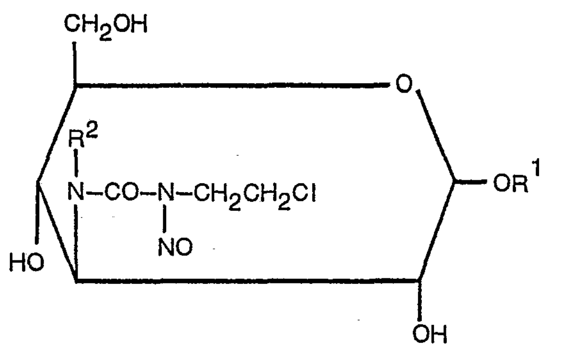

(I)

dans laquelle $R^1$ est un groupe alkyle linéaire ayant un à cinq atomes de carbone et $R^2$ est un groupe alkyle linéaire ou ramifié ayant un à cinq atomes de carbone, un groupe alcényle linéaire ayant deux à cinq atomes de carbone ou méthoxy-alkyle ($C_{1-5}$).

2. Composé selon la revendication 1, dans lequel $R^1$ est un groupe méthyle.

3. Composé selon la revendication 1, dans lequel $R^2$ est le groupe méthyle, n-butyle, isobutyle, allyle ou méthoxyéthyle.

4. Composé selon la revendication 1, 2 ou 3, dans lequel $R^1$ est un groupe méthyle et $R^2$ est un groupe méthyle, n-butyle, isobutyle, allyle ou méthoxyéthyle.

5. Composé selon la revendication 4, dans lequel $R^1$ est un groupe méthyle et $R^2$ est un groupe méthyle.

6. Composé selon la revendication 4, dans lequel $R^1$ est un groupe méthyle et $R^2$ est un groupe isobutyle.

7. Composé selon la revendication 4, dans lequel $R^1$ est un groupe méthyle et $R^2$ est un groupe méthoxyéthyle.

8. Procédé pour la préparation d'un composé de formule:

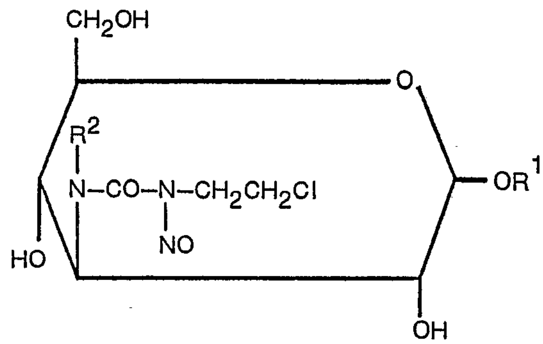

(I)

dans laquelle $R^1$ est un groupe alkyle linéaire ayant un à cinq atomes de carbone, et $R^2$ est un groupe alkyle linéaire ou ramifié ayant un à cinq atomes de carbone, un groupe alcényle linéaire ayant deux à cinq atomes de carbone ou un groupe méthoxy-alkyle ($C_{1-5}$), qui consiste à soumettre un composé de formule:

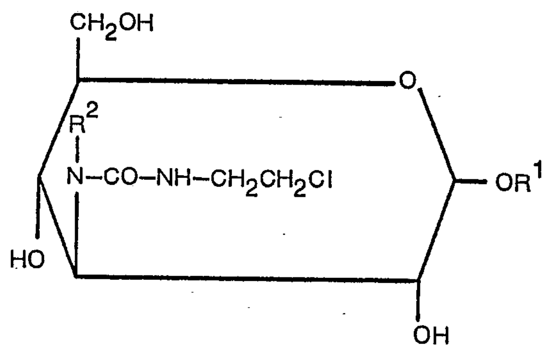

(II)

dans laquelle $R^1$ et $R^2$ ont les mêmes définitions que ci-dessus à une réaction de nitrosation.

9. Procédé selon la revendication 8, dans lequel ladite réaction de nitrosation est effectuée en mettant en contact le composé (II) avec de l'acide nitreux, du trioxyde d'azote ou du tétroxyde d'azote entre −20° et 20°C dans un solvant inerte.

10. Composition thérapeutique qui comprend une quantité thérapeutiquement efficace d'un composé de formule:

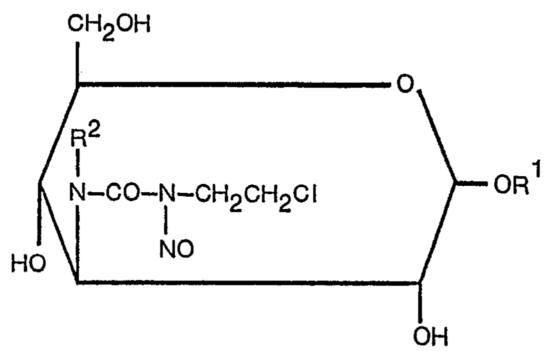

(I)

dans laquelle $R^1$ est un groupe alkyle linéaire ayant un à cinq atomes de carbone, et $R^2$ est un groupe alkyle linéaire ou ramifié ayant un à cinq atomes de carbone, un groupe alcényle linéaire ayant deux à cinq atomes de carbone ou un groupe méthoxy-alkyle ($C_{1-5}$), et un véhicule acceptable pharmaceutiquement pour ce composé.